# EUROPÄISCHE PATENTSCHRIFT

(11) **EP 2 854 877 B1**
(45) Veröffentlichungstag und Bekanntmachung des Hinweises auf die Patenterteilung: **20.09.2023**
(21) Anmeldenummer: 13714860.7
(22) Anmeldetag: 08.04.2013
(51) Int. Cl.: A61L 9/22, A61L 9/20, F24F 8/192, F24F 8/30, B01D 53/32, B01J 19/08

(54) **LUFTREINIGUNGSGERÄT**
AIR CLEANING DEVICE
APPAREIL PURIFICATEUR D'AIR

(30) Priorität: 25.05.2012 DE 102012010342
(43) Veröffentlichungstag der Anmeldung: 08.04.2015
(73) Patentinhaber: AL-KO Therm GmbH, 89343 Jettingen-Scheppach (DE)
(72) Erfinder: DONG, Binjie, 89312 Günzburg (DE); GINSTERBLUM, Stefan, 89358 Kammeltal (DE)
(74) Vertreter: Haseltine Lake Kempner LLP
(86) Internationale Anmeldenummer: PCT/EP2013/001030
(87) Internationale Veröffentlichungsnummer: WO 2013/174467

(56) Entgegenhaltungen:
- EP-A1- 1 479 430
- EP-A1- 1 600 201
- EP-A1- 1 659 665
- WO-A1-00/51714
- WO-A1-2005/061096
- WO-A1-2014/049128
- WO-A2-2006/065491
- WO-A2-2011/149188
- DE-A1-102006 023 069
- US-A1- 2009 324 443

## Beschreibung

Die Erfindung betrifft ein Luftreinigungsgerät zur Reinigung von Luft, insbesondere zur Gebäudeklimatisierung.

Aus DE 10 2006 023 069 A1 ist ein derartiges Luftreinigungsgerät bekannt, das eine Kombination aus einem Plasmagenerator, einem Adsorber und einem Katalysator aufweist. Der Plasmagenerator erzeugt hierbei ein Atmosphärendruckplasma (nicht-thermisches Plasma), das eine entkeimende Wirkung auf die zu reinigende Luft hat. Der Adsorber dient hierbei zum einen zur Adsorption von Verunreinigungen aus der Luft und bildet zum anderen eine Reaktionsoberfläche für chemische Reaktionen. Schließlich ermöglicht der Katalysator in diesem bekannten Luftreinigungsgerät eine katalytische Umwandlung von Verunreinigungen in der Luft.

Der Adsorber und der Katalysator sind hierbei in Form von Schüttgutteilchen zwischen zwei Elektroden des Plasmagenerators angeordnet und werden von der zu reinigenden Luft durchströmt. Das Atmosphärendruckplasma wird hierbei also zwischen den beiden Elektroden des Plasmagenerators innerhalb des Schüttguts erzeugt, das zum einen den Adsorber und zum anderen den Katalysator bildet.

Ein Nachteil dieses bekannten Luftreinigungsgeräts besteht in der Erzeugung von gefährlichen Nebenprodukten im Rahmen der Plasmaerzeugung, was die Verwendbarkeit zur Reinigung von Raumluft einschränkt.

Ein weiterer Nachteil dieses bekannten Luftreinigungsgeräts ist der unbefriedigende Wirkungsgrad bei der Beseitigung von flüchtigen organischen Verbindungen (VOC: Volatile Organic Compounds) und Gerüchen.

Darüber hinaus werden bei dem bekannten Luftreinigungsgerät Mikroorganismen (z.B. Bakterien, Sporen) in der zu reinigenden Luft nicht in vollständig befriedigendem Maße abgetötet.

Schließlich weist das vorstehend beschriebene bekannte Luftreinigungsgerät nur eine unbefriedigende Lebensdauer auf.

Ferner ist zum Stand der Technik hinzuweisen auf US 2004/0234430 A1 und DE 601 11 040 T2. Diese Druckschriften offenbaren jedoch lediglich Abgasreinigungsanlagen zur Reinigung der Abgase von Verbrennungsmotoren und gehören deshalb einem völlig anderen technischen Gebiet an. Derartige Abgasreinigungsanlagen für Verbrennungsmotoren eignen sich auch nicht für die Gebäudeklimatisierung.

Ferner ist zum Stand der Technik hinzuweisen auf WO 2011/149188 A2, DE 10 2006 023069 A1, EP 1 479 430 A1, WO 2006/065491 A2, EP 1 659 665 A1, WO 2005/061096 A1 und WO 00/51714 A1. Diese Druckschriften sind jedoch fernliegend.

Schließlich offenbaren EP 1 600 201 A1 und WO 2014/049128 A1 jeweils ein Luftreinigungsgerät gemäß dem Oberbegriff von Anspruch 1, das jedoch noch nicht vollständig befriedigend ist.

Der Erfindung liegt deshalb die Aufgabe zugrunde, ein entsprechend verbessertes Luftreinigungsgerät zu schaffen.

Diese Aufgabe wird durch ein erfindungsgemäßes Luftreinigungsgerät gemäß dem Hauptanspruch gelöst.

Das erfindungsgemäße Luftreinigungsgerät weist zunächst in Übereinstimmung mit dem Stand der Technik einen Plasmagenerator auf, der ein Plasma erzeugt, um die zu reinigende Luft mit dem Plasma zu behandeln. Bei dem Plasma handelt es sich im Rahmen der Erfindung vorzugsweise um ein Atmosphärendruckplasma, bei welchem der Druck ungefähr dem der umgebenden Atmosphäre, d.h. dem Normaldruck, entspricht. Weiterhin ist zu erwähnen, dass das Plasma vorzugsweise ein nicht-thermisches Plasma ist, bei dem die Elektronen eine viel höhere Temperatur aufweisen als die Schwerteilchen.

Hierbei wird das Plasma im Rahmen der Erfindung durch eine dielektrische Barriereentladung (DBD: Dielectric Barrier Discharge) erzeugt, was an sich aus dem Stand der Technik bekannt ist und deshalb nicht näher beschrieben werden muss. In diesem Zusammenhang ist jedoch zu erwähnen, dass nur eine sogenannte "Volume DBD" in Frage kommt, wobei diese Art der Plasmaerzeugung an sich aus dem Stand der Technik bekannt ist und deshalb nicht näher beschrieben werden muss.

Darüber hinaus weist das erfindungsgemäße Luftreinigungsgerät in Übereinstimmung mit dem Stand der Technik einen Adsorber auf, um Verunreinigungen aus der Luft die adsorbieren und eine Reaktionsfläche für chemische Reaktionen zu bilden. Beispielsweise kann der Adsorber Aktivkohle enthalten, jedoch sind auch andere Materialzusammensetzungen für den Adsorber möglich.

Weiterhin umfasst das erfindungsgemäße Luftreinigungsgerät in Übereinstimmung mit dem Stand der Technik einen Katalysator zur katalytischen Umwandlung von Verunreinigungen in der Luft. Beispielsweise kann der Katalysator Mangandioxid, Kupfer(II)-oxid und/oder Aluminiumoxid enthalten, jedoch sind für den Katalysator auch andere Materialzusammensetzungen möglich.

Das erfindungsgemäße Luftreinigungsgerät zeichnet sich nun gegenüber dem Stand der Technik dadurch aus, dass der Adsorber und der Katalysator zumindest teilweise stromabwärts hinter dem Plasmagenerator angeordnet sind. Im Gegensatz dazu befinden sich der Adsorber und der Katalysator bei dem bekannten Luftreinigungsgerät gemäß DE 10 2006 023 069 A1 zwischen den Elektroden des Plasmagenerators, so dass sich stromabwärts hinter dem Plasmagenerator weder ein Adsorber noch ein Katalysator befindet. Die erfindungsgemäße Anordnung des Adsorbers und des Katalysators zumindest teilweise stromabwärts hinter dem Plasmagenerator ist jedoch vorteilhaft, weil dadurch verhindert wird, dass gefährliche Nebenprodukte der Plasmaerzeugung in die Raumluft gelangen.

Es wurde bereits vorstehend kurz erwähnt, dass der Plasmagenerator das Plasma durch eine volumenbezogene dielektrische Barriereentladung ("Volume DBD") erzeugt. Hierzu weist der Plasmagenerator vorzugsweise zwei Elektroden auf, so dass das Plasma im Volumen zwischen den Elektroden des Plasmagenerators erzeugt wird. Bei einer solchen Anordnung sind der Adsorber und der Katalysator teilweise zwischen den Elektroden des Plasmagenerators und teilweise stromabwärts hinter dem Plasmagenerator angeordnet.

In einem Ausführungsbeispiel der Erfindung weist der Plasmagenerator zur Erzeugung des Plasmas mehrere Elektroden auf, die teilweise luftdurchlässig sind, wobei die zu reinigende Luft entlang einem Strömungsweg durch das Luftreinigungsgerät strömt und der Strömungsweg für die zu reinigende Luft durch die luftdurchlässigen Elektroden des Plasmagenerators hindurchverläuft. Die Luftdurchlässigkeit der Elektroden des Plasmagenerators kann beispielsweise eine Materialeigenschaft des verwendeten Elektrodenmaterials sein oder durch Öffnungen in den ansonsten luftundurchlässigen Elektroden realisiert werden.

In einem bevorzugten Ausführungsbeispiel der Erfindung verläuft der Strömungsweg für die zu reinigende Luft zunächst im Wesentlichen axial durch einen rohrförmigen Strömungskanal und dann im Wesentlichen radial durch eine Wandung des rohrförmigen Strömungskanals hindurch. Die zu reinigende Luft tritt also in axialer Richtung in den rohrförmigen Strömungskanal ein und tritt dann in radialer Richtung wieder aus dem rohrförmigen Strömungskanal aus. Hierbei ist zu erwähnen, dass der Querschnitt des Strömungskanals beliebig gestaltet sein kann, so dass beispielsweise ein runder Querschnitt oder ein eckiger Querschnitt möglich ist. Der im Rahmen der Erfindung verwendete Begriff eines Strömungskanals ist also nicht auf eine bestimmte Querschnittsform des Strömungskanals beschränkt, sondern umfasst beispielsweise auch kreisförmige Querschnitte und rechteckige Querschnitte.

Bei der vorstehend beschriebenen Anordnung mit einem rohrförmigen Strömungskanal können die Elektroden des Plasmagenerators, der Adsorber und/oder der Katalysator jeweils vollständig oder teilweise in der Wandung des Strömungskanals angeordnet sein.

In einer Variante der Erfindung sind der Adsorber und der Katalysator gemischt, wie es beispielsweise auch bei dem Schüttgut gemäß DE 10 2006 023 069 A1 der Fall ist, so dass die Luft gleichzeitig durch den Adsorber und durch den Katalysator strömt.

In einer anderen Variante der Erfindung sind der Adsorber und der Katalysator dagegen in Strömungsrichtung hintereinander angeordnet, insbesondere in mehreren Schichten, die in Strömungsrichtung aufeinander folgen. Hinsichtlich der Reihenfolge von Adsorber und Katalysator entlang des Strömungswegs bestehen im Rahmen der Erfindung zwei Möglichkeiten. Eine Möglichkeit sieht vor, dass der Adsorber stromabwärts hinter dem Katalysator angeordnet ist. Eine andere Möglichkeit sieht dagegen vor, dass der Adsorber stromaufwärts vor dem Katalysator angeordnet ist.

Darüber hinaus können im Rahmen der Erfindung mehrere Adsorber und/oder mehrere Katalysatoren vorgesehen sein.

In einem bevorzugten Ausführungsbeispiel der Erfindung ist zusätzlich ein Ansteuergerät vorgesehen, um den Plasmagenerator elektrisch anzusteuern, wobei das Ansteuergerät den Plasmagenerator alternierend anschaltet und abschaltet, um elektrische Energie zu sparen.

Ferner kann das erfindungsgemäße Luftreinigungsgerät eine UV-Lichtquelle (UV: Ultraviolett) aufweisen, die ultraviolettes Licht zur Bestrahlung der zu reinigenden Luft erzeugt. Hierbei enthält das erfindungsgemäße Luftreinigungsgerät vorzugsweise auch einen Photokatalysator zur Behandlung der Luft, wobei der Photokatalysator dem ultravioletten Licht der UV-Lichtquelle ausgesetzt ist und auf die zu reinigende Luft einwirkt.

Beispielsweise kann ein solcher Photokatalysator Titandioxid oder eine Mischung aus Platin und Titandioxid enthalten, jedoch ist die Erfindung hinsichtlich der Materialzusammensetzung des Photokatalysators nicht auf diese Materialien beschränkt.

Ferner ist noch zu erwähnen, dass sich das erfindungsgemäße Luftreinigungsgerät besonders zur Reinigung von Raumluft oder Außenluft (Zuluft, Abluft und/oder Außenluft) eignet.

In einer Variante der Erfindung saugt das Luftreinigungsgerät Außenluft an, d.h. aus dem Freien. Die angesaugte Außenluft wird dann von dem Luftreinigungsgerät gereinigt. Schließlich wird die gereinigte Außenluft dann in einen Raum eines Gebäudes eingeleitet.

In einer anderen Variante der Erfindung saugt das Luftreinigungsgerät Raumluft aus einem Raum eines Gebäudes an. Die angesaugte Raumluft wird dann von dem Luftreinigungsgerät gereinigt. Schließlich wird die gereinigte Raumluft dann wieder in einen Raum eines Gebäudes eingeleitet.

In einer weiteren Variante der Erfindung saugt das Luftreinigungsgerät ebenfalls Raumluft aus einem Raum eines Gebäudes an. Die angesaugte Raumluft wird dann von dem Luftreinigungsgerät gereinigt. Schließlich wird die gereinigte Raumluft dann nach außen (d.h. ins Freie) geleitet.

Hierbei ist noch zu erwähnen, dass sich das erfindungsgemäße Luftreinigungsgerät zur Luftreinigung von Zuluft, Abluft und/oder Umluft bei verschiedensten Gebäuden eignet, wie beispielsweise Wohnhäusern, Stallgebäuden für Tiere oder Bürogebäuden.

Andere vorteilhafte Weiterbildungen der Erfindung sind in den Unteransprüchen gekennzeichnet oder werden nachstehend zusammen mit der Beschreibung der bevorzugten Ausführungsbeispiele der Erfindung anhand der Figuren näher erläutert. Es zeigen:
- Figur 1A: eine schematische Perspektivansicht eines Luftreinigungsgerätes,
- Figur 1B: eine Querschnittsansicht des Luftreinigungsgeräts aus Figur 1A,
- Figuren 2-13: Querschnittsansichten durch verschiedene, teilweise erfindungsgemäße und teilweise nicht erfindungsgemäße Ausführungsbeispiele eines Luftreinigungsgeräts mit einer Kombination eines Plasmagenerators, eines Adsorbers und eines Katalysators,
- Figur 14: eine schematische Seitenansicht eines nicht erfindungsgemäßen Luftreinigungsgerätes mit einem Plasmagenerator und einer stromabwärts dahinter angeordneten Kombination aus einem Katalysator und einem Adsorber,
- Figur 15: eine nicht erfindungsgemäße Abwandlung von Figur 1 mit einem zusätzlichen Katalysator im Bereich des Plasmagenerators,
- Figur 16: eine nicht erfindungsgemäße Abwandlung von Figur 14 mit einem zusätzlichen Photokatalysator und einer UV-Lichtquelle.

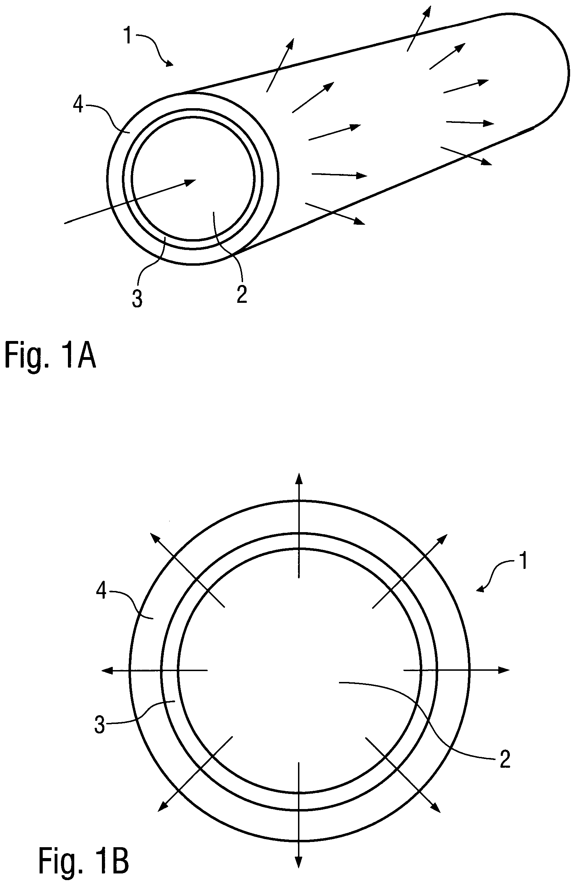

Die Figuren 1A und 1B zeigen eine schematische Darstellung eines erfindungsgemäßen Luftreinigungsgeräts 1, das zur Reinigung von Raumluft eingesetzt werden kann, insbesondere bei der Gebäudeklimatisierung.

Das Luftreinigungsgerät 1 weist einen rohrförmigen Strömungskanal 2 auf, wobei die zu reinigende Luft axial in den Strömungskanal 2 eintritt und dann radial durch die Wandung des Strömungskanals 2 wieder austritt, wie in den Zeichnungen durch Pfeile angedeutet ist, wobei die Pfeile den Strömungsweg der zu reinigenden Luft verdeutlichen. Der rohrförmige Strömungskanal 2 weist deshalb eine Wandung auf, die zumindest teilweise luftdurchlässig ist, damit die zu reinigende Luft radial nach außen durch die Wandung des Strömungskanals 2 hindurchtreten kann.

In der Wandung des rohrförmigen Strömungskanals 2 befindet sich innen ein Plasmagenerator 3, der ein nicht-thermisches Atmosphärendruckplasma erzeugt, um die zu reinigende Luft einer Plasmabehandlung zu unterziehen.

Darüber hinaus befindet sich in der Wandung des rohrförmigen Strömungskanals 2 außen stromwärts hinter dem Plasmagenerator 3 eine Mischung aus einem Adsorber (z.B. Aktivkohle) und einem Katalysator (z.B. Mangandioxid, Kupferdioxid oder Aluminiumoxid), wobei der Adsorber zur Adsorption von Verunreinigungen aus der Luft dient und darüber hinaus eine Reaktionsoberfläche bildet, während der Katalysator eine katalytische Umwandlung von Verunreinigungen in der Luft ermöglicht. Wichtig ist hierbei, dass der Adsorber und der Katalysator in der Wandung des rohrförmigen Strömungskanals 2 stromabwärts hinter dem Plasmagenerator 3 angeordnet sind. Zum einen wird dadurch verhindert, dass gefährliche Nebenprodukte der Plasmaerzeugung in die Raumluft gelangen. Zum anderen wird durch diese Anordnung auch die Wirksamkeit des Adsorbers und des Katalysators erhöht.

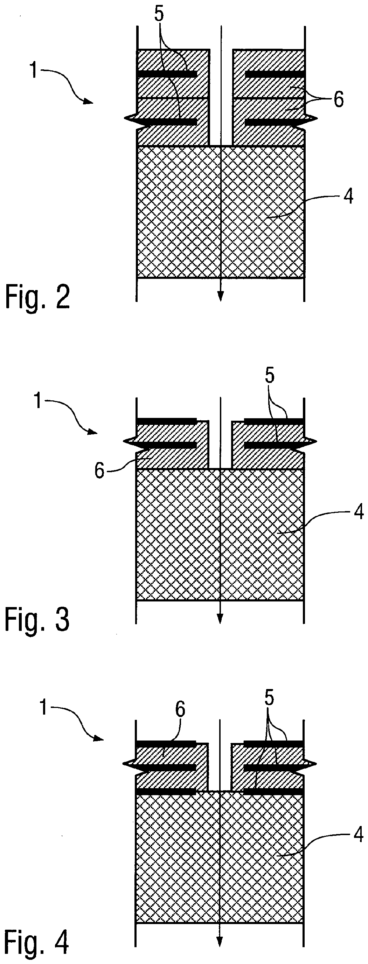

Figur 2 zeigt eine Querschnittsansicht durch die Wandung des rohrförmigen Strömungskanals 2 bei einem nicht erfindungsgemäßen Ausführungsbeispiel des erfindungsgemäßen Luftreinigungsgeräts 1. Der Plasmagenerator 3 weist hierbei mehrere Elektroden 5 und mehrere Dielektrika 6 auf und erzeugt eine oberflächenbezogene dielektrische Barriereentladung ("Surface DBD") .

Die Kombination 4 aus dem Katalysator und dem Adsorber ist hierbei stromabwärts hinter dem Plasmagenerator 3 angeordnet.

Figur 3 zeigt eine Abwandlung des Ausführungsbeispiels gemäß Figur 2, so dass zur Vermeidung von Wiederholungen auf die vorstehende Beschreibung verwiesen wird, wobei für entsprechende Einzelheiten dieselben Bezugszeichen verwendet werden.

Eine Besonderheit dieses nicht erfindungsgemäßen Ausführungsbeispiels besteht darin, dass eine der Elektroden 5 an der Oberfläche des Dielektrikums 6 angeordnet ist, wohingegen die Elektroden 5 bei dem Ausführungsbeispiel gemäß Figur 2 vollständig in das Dielektrikum 6 eingebettet sind.

Figur 4 zeigt eine weitere Abwandlung des Ausführungsbeispiels aus Figur 3, so dass zur Vermeidung von Wiederholungen auf die vorstehende Beschreibung verwiesen wird, wobei für entsprechende Einzelheiten dieselben Bezugszeichen verwendet werden.

Eine Besonderheit dieses nicht erfindungsgemäßen Ausführungsbeispiels besteht darin, dass mehrere Elektroden 5 vorgesehen sind, wobei die Elektroden 5 teilweise in das Dielektrikum 6 eingebettet sind und sich teilweise an der Oberfläche des Dielektrikums 6 befinden.

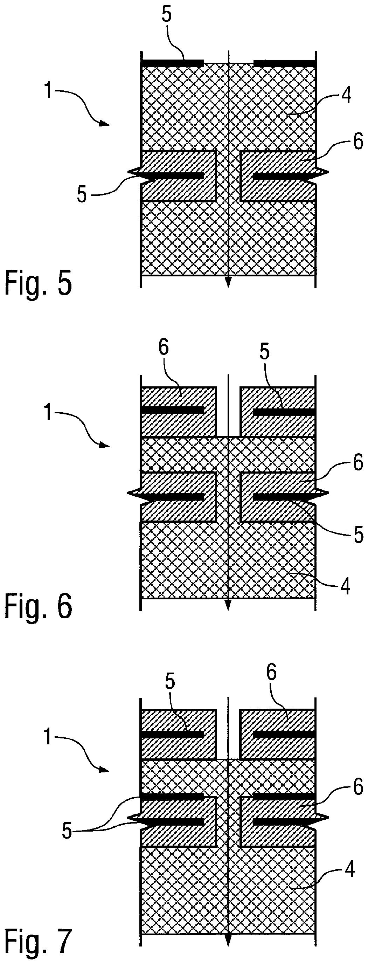

Die Ausführungsbeispiele gemäß den Figuren 5 bis 7 stimmen ebenfalls teilweise mit den vorstehend beschriebenen Ausführungsbeispielen überein, so dass zur Vermeidung von Wiederholungen auf die vorstehende Beschreibung verwiesen wird, wobei für entsprechende Einzelheiten dieselben Bezugszeichen verwendet werden.

Eine Besonderheit dieser Ausführungsbeispiele besteht zunächst darin, dass der Plasmagenerator 3 das Plasma durch eine volumenbezogene dielektrische Barriereentladung ("Volume DBD") erzeugt, wohingegen die Plasmaerzeugung bei den Ausführungsbeispielen gemäß den Figuren 2 bis 4 durch eine oberflächenbezogene dielektrische Barriereentladung ("Surface DBD") erfolgt.

Bei den Ausführungsbeispielen gemäß den Figuren 5 bis 7 befindet sich die Kombination 4 aus dem Adsorber und dem Katalysator deshalb sowohl stromaufwärts vor dem Plasmagenerator 3 als auch stromabwärts hinter dem Plasmagenerator 3 und im Bereich des Plasmagenerators 3.

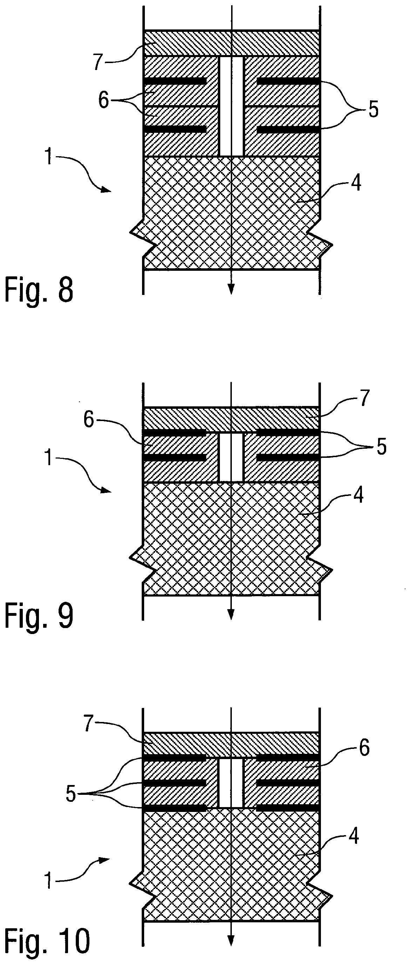

Die Figuren 8 bis 10 zeigen weitere nicht erfindungsgemäße Abwandlungen der Ausführungsbeispiele gemäß den Figuren 2 bis 4, so dass zur Vermeidung von Wiederholungen auf die vorstehende Beschreibung verwiesen wird, wobei für entsprechende Einzelheiten dieselben Bezugszeichen verwendet werden.

Eine Besonderheit dieser nicht erfindungsgemäßen Ausführungsbeispiele besteht darin, dass stromaufwärts vor dem Plasmagenerator 3 und damit auch stromaufwärts vor der Kombination 4 aus Adsorber und Katalysator ein Photokatalysator 7 angeordnet ist, der von einer UV-Lichtquelle (nicht dargestellt) beleuchtet wird und die zu reinigende Luft zusätzlich behandelt, um die Reinigungswirkung zu verbessern.

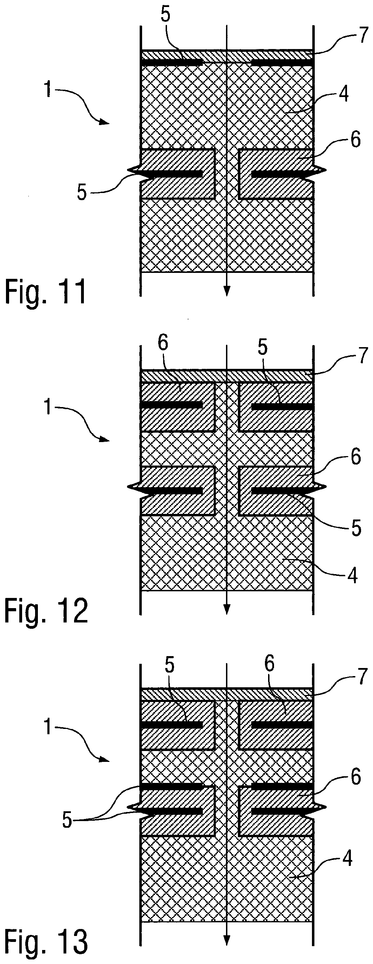

Die Figuren 11 bis 13 zeigen Abwandlungen der Ausführungsbeispiele gemäß den Figuren 5 bis 7, so dass zur Vermeidung von Wiederholungen auf die vorstehende Beschreibung verwiesen wird, wobei für entsprechende Einzelheiten dieselben Bezugszeichen verwendet werden.

Eine Besonderheit dieser Ausführungsbeispiele besteht wiederum darin, dass zusätzlich ein Photokatalysator 7 vorgesehen ist, der von einer UV-Lichtquelle bestrahlt wird, um die Reinigungswirkung durch den Photokatalysator 7 zusätzlich zu verbessern.

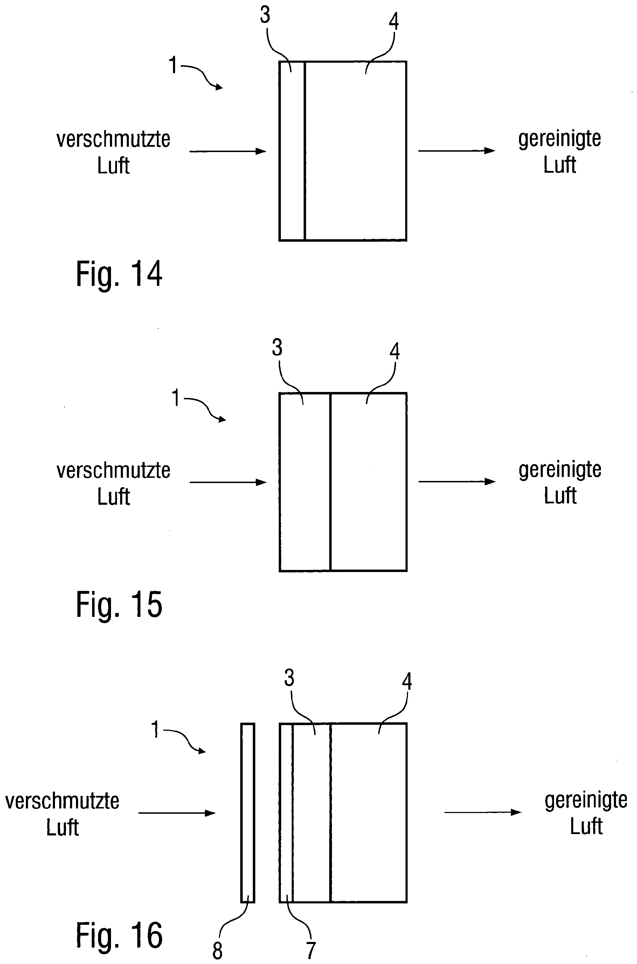

Figur 14 zeigt ebenfalls eine vereinfachte schematische Darstellung eines nicht erfindungsgemäßen Luftreinigungsgerätes 1, so dass zur Vermeidung von Wiederholungen auf die vorstehende Beschreibung verwiesen wird, wobei für entsprechende Einzelheiten dieselben Bezugszeichen verwendet werden.

Hierbei ist die Kombination 4 aus dem Adsorber und dem Katalysator vollständig stromabwärts hinter dem Plasmagenerator 3 angeordnet.

Das nicht erfindungsgemäße Ausführungsbeispiel gemäß Figur 15 stimmt teilweise mit dem Ausführungsbeispiel gemäß Figur 14 überein, so dass zur Vermeidung von Wiederholungen auf die vorstehende Beschreibung verwiesen wird, wobei für entsprechende Einzelheiten dieselben Bezugszeichen verwendet werden.

Eine Besonderheit dieses Ausführungsbeispiels besteht darin, dass im Bereich des Plasmagenerators 3 zusätzlich ein Katalysator angeordnet ist.

Schließlich stimmt auch das nicht erfindungsgemäße Ausführungsbeispiel gemäß Figur 16 teilweise mit den vorstehend beschriebenen Ausführungsbeispielen überein, so dass zur Vermeidung von Wiederholungen auf die vorstehende Beschreibung verwiesen wird, wobei für entsprechende Einzelheiten dieselben Bezugszeichen verwendet werden.

Eine Besonderheit dieses Ausführungsbeispiels besteht darin, dass stromaufwärts vor dem Plasmagenerator 3 ein Photokatalysator 7 angeordnet ist, der von einer UV-Lichtquelle 8 beleuchtet wird, wobei der Photokatalysator 7 die Reinigungswirkung zusätzlich verbessert.

### Bezugszeichenliste:

- 1: Luftreinigungsgerät
- 2: Strömungskanal
- 3: Plasmagenerator
- 4: Kombination aus Adsorber und Katalysator
- 5: Elektroden
- 6: Dielektrika
- 7: Photokatalysator
- 8: UV-Lichtquelle

## Patentansprüche

1. Luftreinigungsgerät (1) mit
a) einem Plasmagenerator (3) zur Behandlung der zu reinigenden Luft mit einem Plasma, insbesondere mit einem Atmosphärendruckplasma,
b) einem Adsorber (4) zur Adsorption von Verunreinigungen aus der Luft und zur Bildung einer Reaktionsoberfläche, und
c) einem Katalysator (4) zur katalytischen Umwandlung von Verunreinigungen in der Luft,
d) wobei der Adsorber (4) und der Katalysator (4) zumindest teilweise stromabwärts hinter dem Plasmagenerator (3) angeordnet sind,
**dadurch gekennzeichnet,**
e) **dass** der Plasmagenerator (3) zur Erzeugung des Plasmas mehrere Elektroden (5) aufweist und im Volumen zwischen den Elektroden (5) eine volumenbezogene dielektrische Barriereentladung erzeugt, und
f) **dass** der Adsorber (4) und der Katalysator (4) teilweise zwischen den Elektroden (5) des Plasmagenerators (3) und teilweise stromabwärts hinter dem Plasmagenerator (3) angeordnet sind.

2. Luftreinigungsgerät (1) nach Anspruch 1, **dadurch gekennzeichnet,**
a) **dass** der Plasmagenerator (3) zur Erzeugung des Plasmas mehrere Elektroden (5) aufweist, und
b) **dass** die Elektroden (5) des Plasmagenerators (3) mindestens teilweise luftdurchlässig sind, und
c) **dass** die zu reinigende Luft entlang einem Strömungsweg durch das Luftreinigungsgerät (1) strömt, und
d) **dass** der Strömungsweg für die zu reinigende Luft durch die luftdurchlässigen Elektroden (5) des Plasmagenerators (3) hindurch verläuft.

3. Luftreinigungsgerät (1) nach Anspruch 2, **dadurch gekennzeichnet, dass** der Strömungsweg für die zu reinigende Luft zunächst axial durch einen rohrförmigen Strömungskanal (2) und dann radial durch eine Wandung des rohrförmigen Strömungskanals (2) verläuft.

4. Luftreinigungsgerät (1) nach Anspruch 3, **dadurch gekennzeichnet, dass** der Strömungskanal einen runden Querschnitt hat.

5. Luftreinigungsgerät (1) nach Anspruch 3, **dadurch gekennzeichnet, dass** der Strömungskanal einen eckigen Querschnitt hat.

6. Luftreinigungsgerät (1) nach einem der Ansprüche 3 bis 5, **dadurch gekennzeichnet, dass** die Elektroden (5) des Plasmagenerators (3) und der Adsorber (4) und der Katalysator (4) vollständig in der Wandung des Strömungskanals (2) angeordnet sind.

7. Luftreinigungsgerät (1) nach einem der vorhergehenden Ansprüche, **dadurch gekennzeichnet, dass** der Adsorber (4) und der Katalysator (4) gemischt sind, so dass die Luft gleichzeitig durch den Adsorber (4) und durch den Katalysator (4) strömt.

8. Luftreinigungsgerät (1) nach einem der Ansprüche 1 bis 6, **dadurch gekennzeichnet, dass** der Adsorber (4) und der Katalysator (4) in Strömungsrichtung hintereinander angeordnet sind, insbesondere in mehreren Schichten, die in Strömungsrichtung aufeinander folgen.

9. Luftreinigungsgerät (1) nach einem der vorhergehenden Ansprüche, **gekennzeichnet durch** ein Ansteuergerät zur elektrischen Ansteuerung des Plasmagenerators (3), wobei das Ansteuergerät den Plasmagenerator (3) alternierend anschaltet und abschaltet, wobei das im angeschalteten Zustand erzeugte Plasma den Adsorber und den Katalysator reaktiviert bzw. reinigt.

## Claims

1. Air cleaning device (1) with
a) a plasma generator (3) for treating the air to be cleaned with a plasma, in particular with an atmospheric pressure plasma,
b) an adsorber (4) for adsorbing impurities from the air and for forming a reaction surface, and
c) a catalytic converter (4) for the catalytic conversion of impurities in the air,
d) wherein the adsorber (4) and the catalytic converter (4) are arranged at least partially downstream of the plasma generator (3),
**characterized**
e) **in that** the plasma generator (3) has a number of electrodes (5) for generating the plasma and generates a volume-related dielectric barrier discharge in the volume between the electrodes (5), and
f) **in that** the adsorber (4) and the catalytic converter (4) are arranged at least partially between the electrodes (5) of the plasma generator (3) and partially downstream of the plasma generator (3).

2. Air cleaning device (1) according to Claim 1, **characterized**
a) **in that** the plasma generator (3) has a number of electrodes (5) for generating the plasma, and
b) **in that** the electrodes (5) of the plasma generator (3) are at least partially air-permeable, and
c) **in that** the air to be cleaned flows along a flow path through the air cleaning device (1), and
d) **in that** the flow path for the air to be cleaned passes through the air-permeable electrodes (5) of the plasma generator (3).

3. Air cleaning device (1) according to Claim 2, **characterized in that** the flow path for the air to be cleaned first passes axially through a tubular flow channel (2) and then passes radially through a wall of the tubular flow channel (2).

4. Air cleaning device (1) according to Claim 3, **characterized in that** the flow channel has a round cross section.

5. Air cleaning device (1) according to Claim 3, **characterized in that** the flow channel has an angular cross section.

6. Air cleaning device (1) according to one of Claims 3 to 5, **characterized in that** the electrodes (5) of the plasma generator (3) and the adsorber (4) and the catalytic converter (4) are arranged completely in the wall of the flow channel (2).

7. Air cleaning device (1) according to one of the preceding claims, **characterized in that** the adsorber (4) and the catalytic converter (4) are combined, so that the air flows simultaneously through the adsorber (4) and through the catalytic converter (4).

8. Air cleaning device (1) according to one of Claims 1 to 6, **characterized in that** the adsorber (4) and the catalytic converter (4) are arranged one behind the other in the direction of flow, in particular in a number of layers that follow one another in the direction of flow.

9. Air cleaning device (1) according to one of the preceding claims, **characterized by** an activation device for electrically activating the plasma generator (3), wherein the activation device alternately activates and deactivates the plasma generator (3), wherein the plasma generated in the activated state reactivates or cleans the adsorber and the catalytic converter.

## Revendications

1. Appareil (1) de purification de l'air comprenant
a) un générateur de plasma (3) pour traiter l'air à purifier avec un plasma, en particulier avec un plasma à pression atmosphérique,
b) un adsorbeur (4) pour adsorber les impuretés de l'air et pour former une surface de réaction, et
c) un catalyseur (4) pour la transformation catalytique des impuretés présentes dans l'air,
d) l'adsorbeur (4) et le catalyseur (4) étant agencés au moins partiellement en aval du générateur de plasma (3),
**caractérisé en ce que**
e) le générateur de plasma (3) présente plusieurs électrodes (5) pour la production du plasma et produit dans le volume entre les électrodes (5) une décharge à barrière diélectrique liée au volume, et
f) l'adsorbeur (4) et le catalyseur (4) sont disposés en partie entre les électrodes (5) du générateur de plasma (3) et en partie en aval du générateur de plasma (3).

2. Appareil de purification d'air (1) selon la revendication 1, **caractérisé en ce que**,
a) le générateur de plasma (3) présente plusieurs électrodes (5) pour la production du plasma, et
b) les électrodes (5) du générateur de plasma (3) sont au moins partiellement perméables à l'air, et
c) l'air à purifier s'écoule le long d'un trajet d'écoulement à travers l'appareil de purification d'air (1), et
d) le trajet d'écoulement pour l'air à purifier passe par les électrodes (5) perméables à l'air du générateur de plasma (3).

3. Appareil de purification d'air (1) selon la revendication 2, **caractérisé en ce que** le trajet d'écoulement pour l'air à purifier s'étend d'abord axialement à travers un canal d'écoulement tubulaire (2) et ensuite radialement à travers une paroi du canal d'écoulement tubulaire (2).

4. Appareil de purification d'air (1) selon la revendication 3, **caractérisé en ce que** le canal d'écoulement a une section transversale circulaire.

5. Appareil de purification d'air (1) selon la revendication 3, **caractérisé en ce que** le canal d'écoulement a une section transversale anguleuse.

6. Appareil de purification d'air (1) selon l'une des revendications 3 à 5, **caractérisé en ce que** les électrodes (5) du générateur de plasma (3) et de l'adsorbeur (4) et le catalyseur (4) sont entièrement agencés dans la paroi du canal d'écoulement (2).

7. Appareil de purification d'air (1) selon l'une quelconque des revendications précédentes, **caractérisé en ce que** l'adsorbeur (4) et le catalyseur (4) sont mélangés de sorte que l'air passe simultanément à travers l'adsorbeur (4) et à travers le catalyseur (4).

8. Appareil de purification d'air (1) selon l'une des revendications 1 à 6, **caractérisé en ce que** l'adsorbeur (4) et le catalyseur (4) sont agencés l'un derrière l'autre dans le sens de l'écoulement, notamment en plusieurs couches qui se suivent dans le sens de l'écoulement.

9. Appareil de purification d'air (1) selon l'une des revendications précédentes, **caractérisé par** un appareil de commande pour la commande électrique du générateur de plasma (3), l'appareil de commande mettant en marche et arrêtant alternativement le générateur de plasma (3), le plasma généré à l'état de marche réactivant ou nettoyant l'adsorbeur et le catalyseur.
